# EUROPEAN PATENT SPECIFICATION

(11) **EP 4 125 833 B1**
(45) Date of publication and mention of the grant of the patent: **07.01.2026**
(21) Application number: 21718958.8
(22) Date of filing: 23.03.2021
(51) Int. Cl.: A61K 31/05, A61K 31/055, A61K 31/09, A61K 31/12, A61P 1/00, A61P 1/04, A61P 1/16, A61P 9/00, A61P 11/00, A61P 13/00, A61P 17/00, A61P 17/06, A61P 19/02, A61P 21/00, A61P 25/28, A61P 27/02, A61P 29/00, A61P 37/00, A61P 37/06, A61P 43/00, C07C 37/14

(54) **INHIBITORS OF THE INTERACTION OF THE UPAR/FPRS RECEPTORS**
INHIBITOREN DER INTERAKTION DER UPAR/FPRS REZEPTOREN
INHIBITEURS DE L'INTERACTION DES RÉCEPTEURS UPAR/FPRS

(30) Priority: 02.04.2020 IT 202000006931
(43) Date of publication of application: 08.02.2023
(73) Proprietor: Orpha Biotech S.r.l., 73024 Maglie (LE) (IT)
(72) Inventor: DE PAULIS, Amato, 73024 Maglie (LE) (IT); LAVECCHIA, Antonio, 73024 Maglie (LE) (IT); MONTUORI, Nunzia, 73024 Maglie (LE) (IT); ROSSI, Francesca Wanda, 73024 Maglie (LE) (IT)
(74) Representative: Fiammenghi, Eva
(86) International application number: PCT/IB2021/052382
(87) International publication number: WO 2021/198844

(56) References cited:
- WO-A2-2006/079929
- WO-A2-2011/014825
- US-A- 2 532 233
- ADELAKUN OLUYEMISI E ET AL: "Enzymatic modification of 2,6-dimethoxyphenol for the synthesis of dimers with high antioxidant capacity", PROCESS BIOCHEMISTRY, vol. 47, no. 12, 18 July 2012 (2012-07-18), pages 1926 - 1932, XP028960391, ISSN: 1359-5113, DOI: 10.1016/J.PROCBIO.2012.06.027
- CHENG-PEI CHUNG ET AL: "Gastroprotective Activities of Adlay (Coix lachryma-jobi L. var. ma-yuen Stapf) on the Growth of the Stomach Cancer AGS Cell Line and Indomethacin-Induced Gastric Ulcers", JOURNAL OF AGRICULTURAL AND FOOD CHEMISTRY, vol. 59, no. 11, 8 June 2011 (2011-06-08), US, pages 6025 - 6033, XP055596199, ISSN: 0021-8561, DOI: 10.1021/jf2009556
- CHOI HYUN GYU ET AL: "Anti-allergic Inflammatory Activities of Compounds of Amomi Fructus", NATURAL PRODUCT COMMUNICATIONS, vol. 10, no. 4, 1 April 2015 (2015-04-01), US, XP093233673, ISSN: 1934-578X, DOI: 10.1177/1934578X1501000425
- V. E. A. REA ET AL: "Discovery of New Small Molecules Targeting the Vitronectin-Binding Site of the Urokinase Receptor That Block Cancer Cell Invasion", MOLECULAR CANCER THERAPEUTICS, vol. 12, no. 8, 22 May 2013 (2013-05-22), US, pages 1402 - 1416, XP055530938, ISSN: 1535-7163, DOI: 10.1158/1535-7163.MCT-12-1249
- SARA L ADAMSKI-WERNER ET AL: "Diflunisal Analogues Stabilize the Native State of Transthyretin. Potent Inhibition of Amyloidogenisis", JOURNAL OF MEDICINAL CHEMISTRY, AMERICAN CHEMICAL SOCIETY, vol. 47, no. 2, 12 November 2003 (2003-11-12), pages 355 - 374, XP002355998, ISSN: 0022-2623
- STEVEN M. JOHNSON ET AL: "Toward Optimization of the Linker Substructure Common to Transthyretin Amyloidogenesis Inhibitors Using Biochemical and Structural Studies +", JOURNAL OF MEDICINAL CHEMISTRY, vol. 51, no. 20, 23 October 2008 (2008-10-23), pages 6348 - 6358, XP055754634, ISSN: 0022-2623, DOI: 10.1021/jm800435s
- KANAME AKAMATA ET AL: "SIRT3 is attenuated in systemic sclerosis skin and lungs, and its pharmacologic activation mitigates organ fibrosis", ONCOTARGET, vol. 7, no. 43, 25 October 2016 (2016-10-25), pages 69321 - 69336, XP055754407, DOI: 10.18632/oncotarget.12504
- C.-K. CHIANG ET AL: "Honokiol, a small molecular weight natural product, alleviates experimental mesangial proliferative glomerulonephritis", KIDNEY INTERNATIONAL, vol. 70, no. 4, 1 August 2006 (2006-08-01), GB, pages 682 - 689, XP055754627, ISSN: 0085-2538, DOI: 10.1038/sj.ki.5001617
- PULIVENDALA GAUTHAMI ET AL: "Honokiol: A polyphenol neolignan ameliorates pulmonary fibrosis by inhibiting TGF-[beta]/Smad signaling, matrix proteins and IL-6/CD44/STAT3 axis both in vitro and in vivo", TOXICOLOGY AND APPLIED PHARMACOLOGY, ACADEMIC PRESS, AMSTERDAM, NL, vol. 391, 4 February 2020 (2020-02-04), XP086074030, ISSN: 0041-008X, [retrieved on 20200204], DOI: 10.1016/J.TAAP.2020.114913
- J. LI ET AL: "Honokiol: an effective inhibitor of tumor necrosis factor- -induced up-regulation of inflammatory cytokine and chemokine production in human synovial fibroblasts", ACTA BIOCHIMICA ET BIOPHYSICA SINICA, vol. 43, no. 5, 20 April 2011 (2011-04-20), US, pages 380 - 386, XP055754647, ISSN: 1672-9145, DOI: 10.1093/abbs/gmr027

## Description

### Field of the art

The present disclosure refers to the medical field and in particular to that of immunology. More in detail the present invention regards a particular class of compounds, suitably selected, for use in the treatment of pathologies arising following phenomena mediated by the interaction of the uPAR/FPRs receptors, as defined in the claims, and typically, but not limited thereto, for use in the treatment of systemic sclerosis. The present invention also refers to pharmaceutical compositions comprising said compounds as well as their use in the treatment of said pathologies, as defined in the claims, and especially in the treatment of systemic sclerosis.

### Prior art

Systemic sclerosis (SSc), also known as Scleroderma, is a multisystemic chronic inflammatory disease of the connective tissue. It is characterized by an altered immune response, production of autoantibodies, giving rise to diffused microvascular disease and fibrosis. The collagen deposit affects not only the skin but also the internal organs, creating anatomic alterations and considerable function deficits. This pathology also indicates considerable problems for the patient who, with the progression of the disease, undergoes phenomena such as the alteration of the face lineaments, the diminution of the function capacity of the hands, the appearance of painful and necrotic skin ulcers, as well alterations of important organ functions.

It is therefore evident that the progressive limitation of the self-sufficiency of the patient renders SSc a pathology which decidedly compromises autonomy and, overall, quality of life. All this also affects the emotional sphere and the psychophysical wellbeing of the subject.

More specifically, this is a pathology which is expressed with progressive thickening and fibrosis of the skin and with specific cases affecting the internal organs (in particular heart, lungs, gastrointestinal tract and kidneys).

The pathogenesis of SSc is up to now largely unknown. In recent decades, considerable progresses have been made in understanding several fundamental aspects of the disease such as the identification of the abnormal production of collagen by the fibroblasts; the extensive alteration of the microcirculation, the endothelial damage and the irregular activation of the immune system. Numerous data confirm the irregularities in each of the aforesaid aspects, however the underlying bases of the disease have not yet been clarified, nor has the irregular initial triggering event been clarified. If follows that, up to now, one single therapy does not exist. In addition, the drugs currently used for the treatment thereof aim for prevention or for treating organ complications ⁽¹⁾.

The studies of the last few years on the pathogenesis have assigned a very important role to the molecular signals mediated by the receptor for urokinase (uPAR) and by the receptors for formylated peptides (FPRs) ⁽²⁾.

uPAR is an anchor-like membrane receptor of GPI type, involved in all the physiological and pathological processes which involve remodeling of the extra-cellular matrix (ECM), such as embryogenesis, inflammation, tissue repair, tumor invasion and metastasis. The traditional role of uPAR is that of bonding a powerful serine-protease, urokinase (uPA), and of focusing its proteolytic activity on the cell surface; however, other functions have also been attributed thereto. Indeed, uPAR also bonds vitronectin (VN), glycoprotein with hepatic synthesis, present in the plasma and abundant in the ECM associated with tumors and inflammatory processes. This bond confers to uPAR the possibility to "grip" on the ECM, thus allowing the required adhesion for the cellular migration. The stimulation of the uPAR, both with uPA and with VN, regulates the adhesion, the migration and the cell proliferation, protects from apoptosis and induces the epithelial-mesenchymal transaction (EMT). In order to perform such activities, uPAR, lacking a transmembrane and cytosolic domain, must interact with the other membrane receptors capable of activating intracellular signaling pathways. It is generally accepted that the main co-receptors of uPAR are integrins, growth factor receptors, such as EGFR and PDGFR, the family of receptors for N-formyl-peptides (fMLF), the FPRs ⁽³⁾.

The FPRs are a family of receptors with innate immunity, coupled to G proteins (GPCR) and involved in the defense of the host against pathogens. The function of FPRs is very well-known in phagocytic leukocytes (e.g. neutrophils and monocytes) which migrate and which are accumulated in the infection sites, where they release ROS and other factors for combatting the invader micro-organisms. The family of FPRs consists of three members: FPR1, FPR2/ALX and FPR3, which have a high sequence homology. FPRs, however, are also expressed in other cell types and in the epithelial tissues. Recently, the involvement of the FPRs was described in the proliferation and repair of the epithelial tissue of intestinal, gastric and nasal type, after inflammatory processes ⁽⁴⁾.

With regard to SSc, the data obtained from the tests executed in the laboratory, in the course of defining the present invention, have indicated that uPAR exerts profibrotic functions through a structural and functional interaction with the FPRs, regulating important processes such as migration, proliferation and production of reactive species of the oxygen in skin fibroblasts obtained from patients affected by SSc. In particular, the studies carried out have allowed a greater understanding of the molecular bases of the disease, demonstrating that the structural and functional inhibition of uPAR with the FPRs, by means of new compounds, can represent a new strategy for SSc therapy ⁽⁵⁾.

On such matter, the studies which have led to the definition of the present invention, described in detail hereinbelow, have been centered on identifying small soluble molecules capable of inhibiting the interaction uPAR/FPRs for the preparation of particular drugs. On such matter, it should be specified that presently the small soluble molecules constitute one of the most attractive therapeutic options both for the bio-availability via oral pathway and for the relatively low production costs.

For the purpose and completion of the following state of the art section of the invention, the documents listed below must be considered.

The US Patent 2,532,233 A, filed by Julius E. Johnson Jr. et al., pertains to a chromatographic device designed for separating substances, thereby improving analytical efficiency. Although not directly related to autoimmune diseases, this technology may be valuable for isolating and characterizing compounds such as 3,3',5,5'-tetramethoxy[1,1'-biphenyl]-4,4'-diol, which could be of interest in pharmacological studies.

The WO 2006/079929 A2 patent describes derivatives of heteroaryl pyridazinone, compounds primarily explored for the treatment of neurological disorders. Their pharmacological properties, particularly for conditions like Alzheimer's disease, suggest potential applications in modulating inflammatory and oxidative processes, which are key factors in autoimmune and fibrotic diseases as well.

Finally, the article by Oluyemisi E. Adelakun et al. ("Enzymatic modification of 2,6-dimethoxyphenol for the synthesis of dimers with high antioxidant capacity," Process Biochemistry, 2012) discusses the enzymatic modification of 2,6-dimethoxyphenol to produce dimers like 3,3',5,5'-tetramethoxy[1,1'-biphenyl]-4,4'-diol. This compound demonstrated a significantly higher antioxidant capacity compared to the original substrate and may have therapeutic applications for diseases characterized by oxidative stress and chronic inflammation, thus representing a promising bioactive compound.

### Description of the invention

The present description regards a particular class of compounds, which in the field of application of the invention fall within the category of the so-called "small molecules", for use as medication.

More in detail the present description refers to a class of compounds selected for a new medical use and especially for use in the treatment of pathologies arising following the start of specific events mediated by the uPAR/FPRs interaction.

Still more in detail, the present description refers to a class of 27 compounds, specifically selected from a pre-existing list, for use in the treatment of pathologies related to the interaction between the aforesaid receptors. The compounds according to the present invention have been elected for use in the treatment of pathologies selected from Systemic sclerosis, Morphea, Eosinophilic fasciitis, Idiopathic pulmonary fibrosis, Glomerulonephritis, Sarcoidosis, Relapsing polychondritis, Rheumatoid arthritis, Systemic Lupus erythematosus, Sjogren's syndrome, Crohn's disease, ulcerative rectocolitis, atrophic antral gastritis, Primary biliary cirrhosis, Polymyositis, Dermatomyositis, Psoriasis, Psoriatic arthritis, Pemphigus, Cutaneous and systemic vasculitis, Uveitis, Behcet's disease, Amyloidosis.

The present description also refers to particular compositions and pharmaceutical formulations, characterized in that they comprise said compounds, and to their use in the treatment of pathologies tied to inflammatory states induced upstream by the structural and functional interaction of uPAR with the FPRs. As an example, for use in the treatment of the aforesaid pathologies and in particular in the treatment of systemic sclerosis. In order to attain the identification of the selected compounds, the research carried out has focused on the functional domain of uPAR, important for the interactions with the FPRs and responsible for numerous biological responses, including adhesion, migration, production of ROS ^{(5,6)}.

Specifically, 27 new compounds were identified by means of similarity searching based on 2D fingerprint and scaffold analysis, in the National Cancer Institute (NCI) Open Database, directed against the SRSY sequence.

The selected inhibitors were characterized *in vitro* by means of assays of protein-protein interaction and cell adhesion on the line of skin fibroblasts BJ (ATCC ^{®} CRL-2522^{™}).

The compounds for use according to the invention and the diseases to be treated by said compounds are as defined in the claims.

### Description of the figures

The invention will be described in detail hereinbelow also with reference to the enclosed figures, in which:

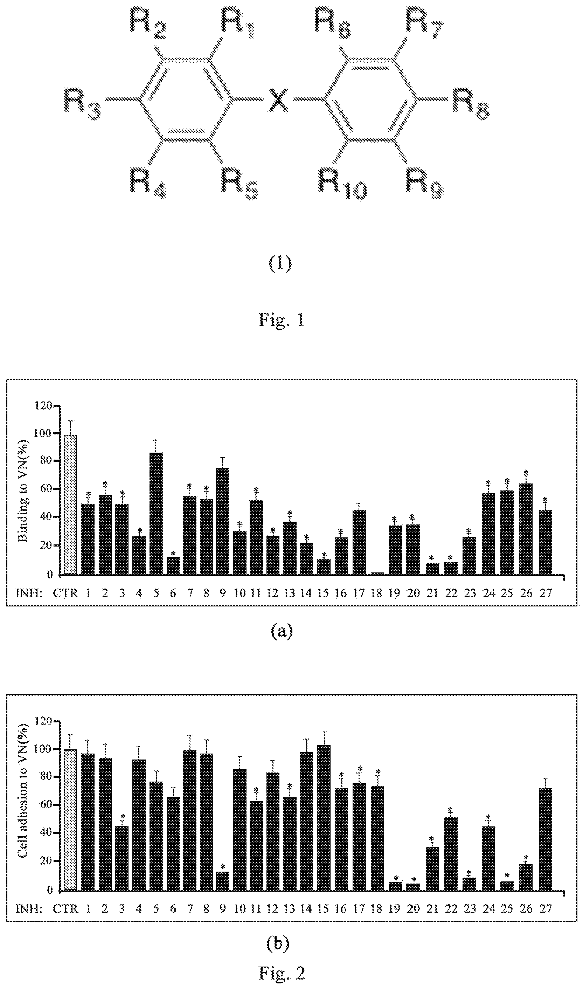

FIGURE 1 shows the general formula that is shared by the 27 selected compounds according to the present invention, for use in the treatment of pathologies tied to uPAR/FPRs interaction.
FIGURE 2 shows the results detected for evaluating the inhibitory activity of the 27 selected compounds on the bond of purified uPAR to the VN in ELISA assays (Fig. 2(A)) and in cell adhesion assays to the VN on BJ cells (Fig. (2B)). Specifically, figure 2(A) shows the percentage of bond to the VN of uPAR, biotinylated in the presence of the compounds at the concentration of 50 µM (black column) or of only the vehicle DMSO (white column), as control. The quantity of uPAR which is bonded to VN in the absence of compounds has been established at 100%. The values represent the mean (± S.D.) of three experiments executed in triplicate (*p<0.05). Figure 2(B) instead shows the percentage of adhesion to the VN of BJ cells treated with the compounds at the concentration of 50µM (black column) or with only the vehicle DMSO (white column). The data represents the mean (±DS) of three independent experiments, each executed in triplicate. The asterisks indicate the statistical significance of the cell adhesion to the VN in the presence of the compounds with respect to the adhesion in the presence of only DMSO, considered equal to 100%, determined by the test t of Student (*p<0.05).

### Detailed description of the invention

In order to identify possible compounds that are structurally and functionally adapted for the inhibition of specific activities mediated by the interaction of the uPAR/FPRs receptors, a silico screening was carried out (similarity searching based on 2D fingerprint and scaffold analysis) in the National Cancer Institute (NCI) Open Database, freely accessible and containing the three-dimensional coordinates of over a quarter million small bio-active drug-like molecules. All this for identifying new analogous structures of the lead compound, 4,4'-*dimethyl[1,1'-biphenyl]-2,2',5,5'-tetraol,* object of a previous study performed by the same research team involved in the definition of the present invention, but provided with an improved inhibitory activity and capable of interfering with the protein-protein interactions of the uPAR-VN or uPAR-FPRs complexes.

It is therefore of interest to specify that the similarity searching in general aims to detect small molecules with similar biological activity, and provides important insight on the structure-activity relations (SAR), thus becoming essential for conducting structural optimization studies ⁽⁷⁾.

Since it is essential to estimate the requirements of the drug-likeness in the initial drug design step (8), the pre-processing of the NCI Open Database was conducted by using the FILTER which reduced the initial database to a subgroup of 164,085 compounds based on the chemical-physical properties and on the functional groups. Then, the subset of drug-like compounds obtained was subjected to a similarity searching based on 2D-fingerprint with the Canvas software (Schrodinger software), using the structure of the lead compound, 4,4'-dimethyl[1,1'-biphenyl]-2,2',5,5'-tetraol as query. In the successive passage, a scaffold analysis allowed selecting only the compounds which had the same molecular scaffold of said compound. In addition, with the objective of expanding the number of chemotypes considered, structurally correlated scaffolds were also included, which maintained the key pharmacophore of 4,4'-dimethyl[1,1'-biphenyl]-2,2',5,5'-tetraol.

The computational strategy employed in the course of defining the object of the invention led to the identification of 27 small molecules (Table 1), which were evaluated for their capacity to inhibit: the bond of uPAR to VN, the cell adhesion mediated by uPAR to VN and the cell migration mediated by uPAR. Compounds 16 and 19 are according to the claimed invention; the other compounds are reference compounds.

**Table 1. List of the substituents for the 27 selected compounds and relative access codes to the NCI Open Database.**

| **Cpd** | **NCI** | **X** | **R₁** | **R₂** | **R₃** | **R₄** | **R₅** | **R₆** | **R₇** | **R₈** | **R₉** | **R₁₀** |
|---|---|---|---|---|---|---|---|---|---|---|---|---|
| **1** | 1267 | absent | H | H | H | H | H | H | H | OH | OH | H |
| **2** | 4187 | absent | H | Me | OH | H | H | H | H | OH | Me | H |
| **3** | 9773 | 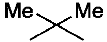 | H | H | Me | H | OH | OH | H | Me | H | H |
| **4** | 11167 | CO | H | H | Cl | H | H | H | Me | OH | H | H |
| **5** | 11186 | CH₂ | H | Me | OH | H | H | H | H | H | H | H |
| **6** | 29020 | 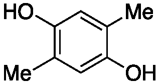 | Me | Me | Me | H | Me | Me | H | OH | Me | Me |
| **7** | 36395 | CH₂ | H | H | H | H | OH | H | H | OH | H | H |
| **8** | 37068 | absent | OH | H | H | H | H | H | H | H | H | OH |
| **9** | 60666 | absent | H | *t*-Bu | OH | Me | H | H | Me | OH | *t*-Bu | H |
| **10** | 128399 | absent | H | Me | OH | Me | H | H | Me | OH | Me | H |
| **11** | 135414 | absent | H | Et | OH | Me | H | H | Me | OH | Et | H |
| **12** | 407988 | absent | H | H | H | H | H | OH | H | H | OH | H |
| **13** | 408489 | 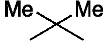 | H | Me | OH | H | H | H | Me | OH | H | H |
| **14** | 65069 | absent | OH | H | OH | H | OH | H | OH | H | OH | H |
| **15** | 66167 | absent | H | H | OH | H | OH | OH | H | OH | H | H |
| **16** | 83599 | absent | H | OMe | OH | OMe | H | H | OMe | OH | OMe | H |
| **17** | 133369 | absent | H | OH | OH | H | H | H | H | OH | OH | H |
| **18** | 143541 | absent | H | OH | OH | OH | H | H | OH | OH | OH | H |
| **19** | 2817 | absent | H | Pr | OH | H | H | H | H | OH | Pr | H |
| **20** | 7781 | CH₂ | OH | *t*-Bu | H | Me | H | OH | *t*-Bu | H | Me | H |
| **21** | 7782 | CH₂ | H | Et | H | *t*-Bu | OH | OH | *t*-Bu | H | Et | H |
| **22** | 29013 | 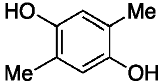 | H | H | Me | H | Me | Me | H | Me | H | H |
| **23** | 35255 | CH₂ | H | *t*-Bu | H | Cl | OH | OH | Cl | H | *t*-Bu | H |
| **24** | 57617 | absent | H | Allyl | OH | H | H | H | H | OH | Allyl | H |
| **25** | 67513 | CH₂ | H | *t*-Bu | OH | Me | H | H | *t*-Bu | OH | Me | H |
| **26** | 212511 | absent | Me | H | Me | H | Me | OH | H | Me | OH | H |
| **27** | 10369 | absent | H | H | F | H | OMe | OMe | H | F | H | H |

**Table 2. Nomenclature of the 27 selected compounds**

| **Cpd** | **Chemical name** |
|---|---|
| **1** | [1,1'-biphenyl]-3,4-diol |
| **2** | 3,3'-dimethyl[1,1'-biphenyl]-4,4'-diol |
| **3** | 6,6'-(propan-2,2-diyl)bis(3-methylphenol) |
| **4** | (4-chlorophenyl)(4-hydroxy-3-methylphenyl)methanone |
| **5** | 4-benzyl-2-methylphenol |
| **6** | 2,2",3,3",4",6,6"-heptamethyl-[1,1':4',1''-terphenyl]-2',4,5'-triol |
| **7** | *o*-(*p*-hydroxybenzyl)phenol |
| **8** | [1,1'-biphenyl]-2,2'-diol |
| **9** | 3,3'-ditert-butyl-5,5'-dimethyl[1,1'-biphenyl]-4,4'-diol |
| **10** | 3,3',5,5'-tetramethyl[1,1'-biphenyl]-4,4'-diol |
| **11** | 3,3'-diethyl-5,5'-dimethyl[1,1'-biphenyl]-4,4'-diol |
| **12** | [1,1'-biphenyl]-2,5-diol |
| **13** | 4,4'-(propan-2,2-diyl)bis(2-methylphenol) |
| **14** | [1,1'-biphenyl]-2,3',4,5',6-pentol |
| **15** | [1,1'-biphenyl]-2,2',4,4'-tetrol |
| **16** | 3,3',5,5'-tetramethoxy[1,1'-biphenyl]-4,4'-diol |
| **17** | [1,1'-biphenyl]-3,3',4,4'-tetrol |
| **18** | [1,1'-biphenyl]-3,3',4,4',5,5'-hexol |
| **19** | 3,3'-dipropyl[1,1'-biphenyl]-4,4'-diol |
| **20** | 6,6'-methylenebis(2-(tert-butyl)-4-methylphenol) |
| **21** | 6,6'-methylenebis(2-(tert-butyl)-4-ethylphenol) |
| **22** | 2,2'',4,4''-tetramethyl-[1,1':4',1''-terphenyl]-2',5'-diol |
| **23** | 6,6'-methylenebis(4-(tert-butyl)-2-chlorophenol) |
| **24** | 3,3'-diallyl[1,1'-biphenyl]-4,4'-diol |
| **25** | 4,4'-methylenebis(2-(tert-butyl)-6-methylphenol) |
| **26** | 2',4,4',6'-tetramethyl[1,1-biphenyl]-2,5-diol |
| **27** | 4,4-difluoro-2,2'-dimethoxy-1,1'-biphenyl |

According to the present disclosure, a compound of general formula was therefore identified like that indicated in figure 1, i.e. compound of formula 1, in which different possible substituents present therein, suitably identified, confer to said compound the aforesaid searched inhibitory activity.

Specifically, the possible substituents are such that for R1, R2, R3, R4, R5, R6, R7, R8, R9 and R10, these are selected in the group consisting of: H, Me, OH, t-Bu, Et, OMe, Pr, Allyl, Cl, F.

Preferably possible substituents for R1 are selected in the group consisting of: H, Me, OH; for R2 are selected in the group consisting of: H, OH, Me, t-Bu, Et, OMe, Pr, Allyl; for R3 are selected in the group consisting of: H, OH, Me, Cl, F; for R4 are selected in the group consisting of: H, Me, OMe, *t*-Bu, Cl; for R5 are selected in the group consisting of: H, OH, Me, OMe, for R6 are selected in the group consisting of: H, Me, OH, OMe; for R7 are selected in the group consisting of: H, Me, OMe, *t*-Bu, Cl, for R8 are selected in the group consisting of: OH, Me, H, F; per R9 are selected in the group consisting of; OH, Me, H, *t-*Bu, Et, OMe, Pr, Allyl; for R10 are selected in the group consisting of: H, Me, OH; and in X said substituent is: either absent or is selected from the group consisting of: 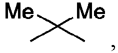 CO, CH₂,

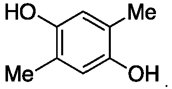

Still more preferably, the following combination and selection of the substituents have led to the definition of 27 selected compounds corresponding to the 27 compounds listed in table 1. Specifically the preferred compounds are those having formula 1 and in which specific substituents are assigned via R1, R2, R3, R4, R5, R6, R7, R8, R9, R10, while for X a substituent can be selected from among those listed above or said substituent may be absent (in table 1, indication corresponding to X is absent).

With reference to figure 2 and to tables 1 and 2, the compounds identified as more suitable in the treatment of the pathologies that follow events mediated by the uPAR/FPRs interaction, are the following: the compounds 3, 11, 13, 16, 17, 18, 19, 20, 21, 22, 23, 24, 25, 26 indicated in the tables 1 and 2, i.e. the compounds:
6,6'- (propan-2,2-diyl)bis(3-methylphenol);
3,3'-diethyl-5,5'-dimethyl[1,1'-biphenyl]-4,4'-diol;
4,4'-(propan-2,2-diyl)bis(2-methylphenol),
3,3',5,5'-tetramethoxy[1,1'-biphenyl]-4,4'-diol;
[1,1'-biphenyl]-3,3',4,4'-tetrol;
[1,1'-biphenyl]-3,3',4,4',5,5'-hexol;
3,3'-dipropyl[1,1'-biphenyl]-4,4'-diol;
6,6'-methylenebis(2-(*tert*-butyl)-4-methylphenol);
6,6'-methylenebis(2-(*tert*-butyl)-4-ethylphenol);
2,2'',4,4''-tetramethyl-[1,1':4',1''-terphenyl]-2',5'-diol;
6,6'-methylenebis(4-(*tert*-butyl)-2-chlorophenol);
3,3'-diallyl[1,1'-biphenyl]-4,4'-diol;
4,4'-methylenebis(2-(*tert*-butyl)-6-methylphenol);
2',4,4',6'-tetramethyl[1,1'-biphenyl]-2,5-diol.
3,3',5,5'-tetramethoxy[1,1'-biphenyl]-4,4'-diol and 3,3'-dipropyl[1,1'-biphenyl]-4,4'-diol are according to the invention; the other compounds are reference compounds.

It is of interest to specify that most of said selected compounds are commercially available or in any case can be synthesized with procedures and methods that are known to the man skilled in the art.

Still with reference to figure 2, the conducted experiments have indicated that, among said compounds, those which showed greater inhibitory activity against the interaction of the uPAR with the VN *in vitro* and against its cell adhesion to the VN, are the compounds 3, 19, 20, 23 indicated in said tables 1 and 2. In particular those most promising are the compounds 19, 20 and 23, for which a considerable contribution was encountered to the aforesaid inhibition of the cell adhesion.

Said compounds are specifically 3,3'-dipropyl[1,1'-biphenyl]-4,4'-diol; 6,6'-methylenebis(2-(tert-butyl)-4-methylphenol); 6,6'-methylenebis(4-(tert-butyl)-2-chlorophenol).

Still more specifically the structure of said compounds corresponds to that of the compound of formula 1
- in which: X is absent; R1 = H; R2 = Pr; R3 = OH; R4 = H; R5 = H; R6 =H; R7 = H; R8 = OH; R9 = Pr: R10 = H (compound 19; according to the invention); or
- X = CH₂; R1 = OH R2 = t-Bu R3 = H; R4 = Me; R5 = H; R6 = OH; R7 = t-Bu; R8 = H R9 = Me; R10 = H (reference); or
- X = CH₂; R1 = H; R2 = t-Bu; R3 = H; R4 = Cl; R5 = OH; R6 = OH; R7 = Cl; R8 =H; R9 = t-Bu; R10 = H (reference).

### Materials and methods

### Computational Chemistry

### Preparation of the ligand database

The NCI Open Database, containing 265,242 ligands, was processed with the Filter software (release 3.1.1.2, OpenEye Scientific Software Inc., Santa Fe, USA, http://www.eyesopen.com/) in order to remove molecules with undesired chemical-physical properties such as poor oral bioavailability, non-standard chemical elements, excessively reactive functional groups. The resulting database consisted of 164,085 structures with drug-like characteristics.

### Similarity searching based on 2D fingerprint and scaffold analysis

The fingerprint MACCS ⁽⁹⁾, implemented in the Canvas software (Schrödinger Release 2019-3: Canvas, Schrödinger, LLC, New York, NY, 2019.) was employed for coding the structural characteristics of the compounds contained in the database. The fingerprint MACCS consists of a dictionary of predefined structural fragments. The Tanimoto coefficient (Tc), which is widely used in the scope of similarity searching ⁽¹⁰⁾, was selected for quantifying the similarity between the lead compound, *4,4'-dimethyl[1,1'-biphenyl]-2,2',5,5'-tetraol,* and the compounds present in the database, assigning a score comprised between 0 (if the compounds do not have fragments in common) and 1 (if the compounds have identical fragments). Subsequently, the compounds of the database were ordered in decreasing order with reference to the score and the first 1640 compounds (which represent 1% of the entire database) were selected for further research. Indeed, it was reported in preceding studies that the pre-selection of a subset of structures of such order of magnitude (~1%) from a database allows on average recovering 25% of the scaffolds and hence obtaining a greater number of structurally different hits ⁽¹¹⁾. Subsequently, the hits were analyzed by means of the scaffold decomposition module implemented in Canvas. 100 compounds were collected that contain the biphenyl scaffold of the lead compound *4,4'-dimethyl[1,1'-biphenyl]-2,2',5,5'-tetraol,* also including several congeners which contained this structural motif. Finally, based on the chemical intuition and on the availability of the compounds, we selected and ordered 27 molecules for the biological tests.

### Preparation of the protein

The coordinates of the crystalline structure of the ternary complex between the receptor uPAR, the N-terminal (ATF) fragment of urokinase (uPA) and the somatomedin B (SMB) domain of vitronectin were downloaded from the Protein Data Bank (PDB code: 3BT1) ⁽¹²⁾. The unresolved segment comprised between the residues 82-85 was constructed by means of the graphical interface of the MODELLER software in Chimera 1.6.1 ⁽¹³⁾. The structure of the protein was then prepared by means of the module "Protein Preparation" of the graphical interface of Maestro. The hydrogen atoms were added to the protein, considering the physiological pH (7.0). The obtained structure was then energetically minimized by means of 2500 iterations by using the method of the Polak-Ribiere conjugate gradient and the force field OPLS3, implemented in Macromodel, in order to eliminate possible inconsistencies in the bond geometries.

The compounds, identified by means of the similarity searching and the subsequent biological assays, were constructed with the Molecular Builder module in Maestro and prepared for docking by means of the LigPrep software (LigPrep, Schrödinger, LLC, New York, NY, 2019). The resulting structures were optimized with Macromodel (Schrödinger, LLC, New York, NY, 2019), by using the force field MMFF with the steepest descent method (1000 steps) followed by 500 steps with the conjugate gradient method of truncated-Newton type. The partial atomic loads were assigned with the force field OPLS-AA.

### Molecular Docking

The docking simulations of the compounds identified via similarity in the bond site of the VN on uPAR were carried out with the genetic algorithm implemented in GOLD 5.5 (CCDC Software Limited, Cambridge, UK, 2008) ⁽¹⁴⁾. For the docking simulations, a grid was calculated, centered on the residues involved in the bond with the VN within a range of 10 Å, employing the default parameters of the program. The chemotactic sequence SRSRY (comprised between the residues 88-92), situated on the mobile loop that connects the domains DI and DII of uPAR, was considered flexible. The mobility of such residues was set by means of a specific option in GOLD, which implements the Lovell library of rotamers ⁽¹⁵⁾. The poses obtained were classified based on the scoring functions ChemPLP and ChemScore ⁽¹⁶⁾. The ligand-final receptor complex for each compound was selected by selecting the pose with the best score, consistent with the experimentally-derived information.

Based on the obtained results, pharmaceutical compositions and their possible formulations were designed and prepared, comprising the compounds selected for use in the treatment of pathologies related to the uPAR/FPRs interaction, as an example for use in the treatment of pathologies such as: Systemic sclerosis, Morphea, Eosinophilic fasciitis, Idiopathic pulmonary fibrosis, Glomerulonephritis, Sarcoidosis, Relapsing polychondritis, Rheumatoid arthritis, Systemic Lupus erythematosus, Sjogren's syndrome, Crohn's disease, ulcerative rectocolitis, Atrophic antral gastritis, Primary biliary cirrhosis, Polymyositis, Dermatomyositis, Psoriasis, Psoriatic arthritis, Pemphigus, Cutaneous and systemic vasculitis, Uveitis, Behcet's disease, Amyloidosis, and especially for use in the treatment of systemic sclerosis.

Object of the present description is also a pharmaceutical composition comprising at least one of the following compounds selected from among:
6,6'- (propan-2,2-diyl)bis(3-methylphenol);
3,3'-diethyl-5,5'-dimethyl[1,1'-biphenyl]-4,4'-diol,
4,4'-(propan-2,2-diyl)bis(2-methylphenol);
3,3',5,5'-tetramethoxy[1,1'-biphenyl]-4,4'-diol;
[1,1'-biphenyl]-3,3',4,4'-tetrol;
[1,1'-biphenyl]-3,3',4,4',5,5'-hexol;
3,3'-dipropyl[1,1'-biphenyl]-4,4'-diol;
6,6'-methylenebis(2-(*tert*-butyl)-4-methylphenol);
6,6'-methylenebis(2-(*tert*-butyl)-4-ethylphenol);
2,2'',4,4''-tetramethyl-[1,1':4',1''-terphenyl]-2',5'-diol;
6,6'-methylenebis(4-(*tert*-butyl)-2-chlorophenol);
3,3'-diallyl[1,1'-biphenyl]-4,4'-diol;
4,4'-methylenebis(2-(*tert*-butyl)-6-methylphenol);
2',4,4',6'-tetramethyl[1,1'-biphenyl]-2,5-diol;
or combinations thereof, and its use in medicine and in particular in the treatment of said pathologies: Systemic sclerosis, Morphea, Eosinophilic fasciitis, Idiopathic pulmonary fibrosis, Glomerulonephritis, Sarcoidosis, Relapsing polychondritis, Rheumatoid arthritis, Systemic Lupus erythematosus, Sjogren's syndrome, Crohn's disease, ulcerative rectocolitis, Atrophic antral gastritis, Primary biliary cirrhosis, Polymyositis, Dermatomyositis, Psoriasis, Psoriatic arthritis, Pemphigus, Cutaneous and systemic vasculitis, Uveitis, Behcet's disease, Amyloidosis and, typically, but not limited thereto, in the treatment of systemic sclerosis. Preferably said composition comprises a selection of the following: 3,3'-dipropyl[1,1'-biphenyl]-4,4'-diol; 6,6'-methylenebis(2-(tert-butyl)-4-methylphenol); 6,6'-methylenebis(4-(tert-butyl)-2-chlorophenol), or combinations thereof, and pharmaceutically acceptable excipients, as an example solvents such as alcohols or organic solvents, possibly in combination, light liquid paraffin dispersed in an aqueous medium by means of surfactants, mineral oil, wax. Said composition can be formulated as: solutions, suspensions, powders, granules, tablets, pills, capsules, syrups, suppositories, sprays, aerosols or controlled-release systems, cream, ointment or gel, lubricants, pastes, syrups, vials, drops, eye drops, aerosols that are acceptable for medical use. The compositions can be administered through different pathways, in particular orally, transdermally, subcutaneously, intravenously, intramuscularly, rectally and intranasally.

### Chemical inhibitors

The selected compounds were obtained from the Open Chemical Repository of the Developmental Therapeutics Program (DTP) at the National Cancer Institute (NCI) (http://dtp.cancer.gov). Subsequently, the lyophilized compounds were resuspended in dimethyl sulfoxide (DMSO) at a concentration of 0.01 mol/L and preserved at -20 ° C.

### Cell cultures

The cell line BJ (ATCC CRL2522) was used as model of human skin fibroblasts. The cells were cultivated in Dulbecco's Modified Eagle Medium (DMEM) containing fetal bovine serum at 10% (FBS) at 37° C with CO₂ at 5%.

### Biotinylation of uPAR

Soluble recombinant uPAR (suPAR; R&D System, Minneapolis, MN, USA) was biotinylated and purified with the biotinylation module of the proteins ECL Amersham according to the producer's instructions (GE Healthcare, Buckinghamshire, UK).

### Bond of soluble uPAR to immobilized VN

The (high-binding) plates with 96 wells (Corning, New York, USA) were covered with 0.5 µg/well of VN (Becton Dickinson Biosciences, Franklin Lakes, NJ, USA) diluted in PBS (0.08 M NaCl, 0.002 M KCl, 0.0115 M Na₂HPO₄, 0.002 M KH₂PO₄, pH 7.2) or bovine serum albumin (BSA), as negative control, and incubated at 4° C for the entire night. After a washing in PBS, the residual bond sites were blocked with 200 µL of BSA at 1% in PBS, for 1 hour at ambient temperature. 25 nmol/L of biotinylated s-uPAR (diluted in PBS, 1 mg/mL of BSA), on its own or in the presence of selected compounds, were placed in the covered wells. The plates were maintained at 4° C for 1 hour, washed with PBS containing 0.1% Tween 20 and then avidin marked with peroxidase (Amersham), diluted 1:1.500 in PBS containing 10 mg/mL of BSA. After further washings, the substrate of peroxidase o-phenylenediamine (Sigma, St. Louis, MO, USA) was added and left to react for 3 minutes. The reactions were stopped with 1 mol/L H₂SO₄ and the product was measured at 492 nm by using an automated plate reader (Bio-Rad, Munich, Germany).

### Assay of cell adhesion

The plates with 96 wells (Nunc, Roskilde, Denmark) were covered with VN (1 µg) or with 100 µl of 1% BSA deactivated with heat in PBS, as negative control, and incubated during the entire night at 4° C. The plates were then blocked 1 hour at ambient temperature with 1% BSA deactivated with heat in 1% PBS. The cells were detached with PBS containing 2 mM of EDTA and washed with DMEM lacking serum, they were counted, plated in the wells with a density of 10⁵ cells/well and incubated for 1 hour at 37° C in the presence of or in the absence of the selected compounds. The cells were then fixed with paraformaldehyde at 3% in PBS for 10 minutes and then incubated with methanol at 2% for 10 minutes. The cells were finally colored for 10 minutes with 0.5% crystal violet in 20% methanol, eluted with 0.1 mol/l of sodium citrate in 50% ethanol (pH 4.2) and the absorbance was measured at 540 nm with a spectrophotometer.

The invention, as defined in the appended claims, is described in detail in the present document. However, all technical features and boundaries of the invention are exclusively those defined in the claims.

### Bibliography

1 Gabrielli A, Avvedimento EV, Krieg T. Scleroderma. N Engl JMed (2009) 360:1989-2003*.*
2 Rossi FW, Napolitano F, Pesapane A, Mascolo M, Staibano S; Matucci-Cerinic M, et al. Upregulation of the N-Formyl Peptide Receptors in Sclerodenna Fibroblasts Fosters the Switch to Myofibroblasts. J Immunol (2015) 194:5161-73*.*
3 Montuori N, Ragno P. Multiple activities of a multifaceted receptor: roles of elevated and soluble uPAR. Front Biosci (2009) 14:2494-503*.*
4 Prevete N, Liotti F, Marone G, Melillo RM, de Paulis A. Formyl peptide receptors at the interface of inflammation, angiogenesis and tumor growth. Pharmacol Res; (2015) 102:184-91*.*
5 Rea VE Lavecchia A, Di Giovanni C, Rossi FW, Gorrasi A, Pesapane A, et al. Discovery of New Small Molecules Targeting the Vitronectin-Binding Site of the Urokinase Recepto That Block Cancer Cell Invasion. Mol Cancer Ther (2013) 12:1402-16*.*
6 Napolitano F, Rossi FW, Pesapane A, Varricchio S, Ilardi G, Mascolo M et al. N-Formyl Peptide Receptors Induce Radical Oxygen Production in Fibroblasts Derived From Systemic Sclerosis by Interacting With a Cleaved Form of Urokinase Receptor. Froni Immunol (2018) 9:574*.*
7 *a)* Stumpfe D and Jürgen B. Similarity searching. Wiley Interdisciplinary Reviews: Comput Mol Sci (2011) 1:260-282*. b)* Lavecchia A and Di Giovanni C. Virtual screening strategies in drug discovery: a critical review. Current medicinal chemistry (2013) 20:232839-2860*.*
8 Lipinski CA, Lombardo F, Dominy BW, Feeney PJ. Experimental and computational approaches to estimate solubility and permeability in drug discovery and development settings. Adv Drug Deliv Rev (2001) 46:3-26*.*
9 Brown RD and Martin YC. Use of Structure-Activity Data to Compare Structure- Based Clustering Methods and Descriptors for Use in Compound Selection, J Chem Inf Comput Sci (1996) 36: 572-584*.,*
10 Willett P. Similarity searching using 2D structural fingerprints. Methods Mol Biol (2011) 672:133-158*.,*
11 Vogt M, Stumpfe D, Geppert H, Bajorath J. Scaffold hopping using two-dimensional fingerprints: true potential, black magic, or a hopeless endeavor? Guidelines for virtual screening. J. Med. Chem. (2010) 53:5707-5715*.*
12 Huai Q, Zhou A, Lin L, Mazar AP, Parry GC, Callahan J, et al. Crystal structures of two human vitronectin, urokinase and urokinase receptor complexes. Nat Struct Mol Biol (2008) 15:422-3*.*
13 Yang Z, Lasker K, Schneidman-Duhovny D, Webb B, Huang CC, Pettersen, EF, et al. UCSF Chimera, MODELLER, and IMP: an integrated modeling system. J Struc Biol (2012) 179:269-278*.*
14 Jones G, Willett P, Glen RC, Leach AR, Taylor R. Development and validation of a genetic algorithm for flexible docking. J Mol Biol (1997) 267:727-748*.*
15 Lovell SC, Word JM, Richardson JS, Richardson DC. The Penultimate Rotamer Library. Richardson, Proteins (2000) 40:389-408*.*
16 Verdonk ML, Cole JC, Hartshorn MJ, Murray CW, Taylor RD. Improved protein-ligand docking using GOLD. Proteins Struct Funct Bioinf (2003) 52:609-623*.*

## Claims

1. Compound of formula (1) 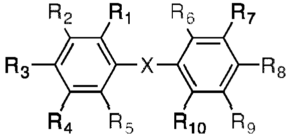 **wherein**
X is absent; R1 = H; R2 = Pr; R3 = OH; R4 = H; R5 = H; R6 =H; R7 = H; R8 = OH; R9 = Pr: R10 = H for use in the treatment of diseases selected from the group consisting of: Systemic sclerosis, Morphea, Eosinophilic fasciitis, Idiopathic pulmonary fibrosis, Glomerulonephritis, Sarcoidosis, relapsing polychondritis, rheumatoid arthritis, systemic Lupus erythematosus, Sjogren's syndrome, Crohn's disease, ulcerative rectocolitis, atrophic antral gastritis, primary biliary cirrhosis, polymyositis, dermatomyositis, Psoriasis, psoriatic arthritis, pemphigus, cutaneous and systemic vasculitis, uveitis, Behcet's Disease, Amyloidosis.

2. Pharmaceutical composition comprising 3,3',5,5'-tetramethoxy[1,1'-biphenyl]-4,4'-diol; and pharmaceutically acceptable excipients thereof for use in the treatment of diseases selected from the group consisting of: Systemic sclerosis, Morphea, Eosinophilic fasciitis, Idiopathic pulmonary fibrosis, Glomerulonephritis, Sarcoidosis, relapsing polychondritis, rheumatoid arthritis, systemic Lupus erythematosus, Sjogren's syndrome, Crohn's disease, ulcerative rectocolitis, atrophic antral gastritis, primary biliary cirrhosis, polymyositis, dermatomyositis, Psoriasis, psoriatic arthritis, pemphigus, cutaneous and systemic vasculitis, uveitis, Behcet's Disease, Amyloidosis.

3. Pharmaceutical composition for use according to claim 2 or pharmaceutical composition comprising the compound of claim 1 for use according to claim 1, in the form of acceptable solutions, suspensions, powders, granules, tablets, pills, capsules, syrups, suppositories, sprays, aerosols or controlled-release systems, cream, ointment or gel, lubricants, pastes, syrups, vials, drops, eye drops, aerosols.

4. Pharmaceutical composition for use according to claim 2 or pharmaceutical composition comprising the compound of claim 1 for use according to claim 1, to be administered orally, transdermally, subcutaneously, intravenously, intramuscularly, rectally and intranasally.

## Patentansprüche

1. Verbindung der Formel (I) 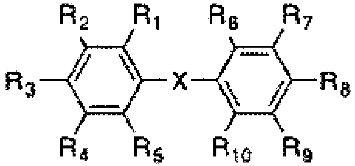 **wobei**
X fehlt; RI = H; R2 = Pr; R3 = OH; R4 = H; RS = H; R6 = H; R7 = H; R8 = OH; R9 = Pr; RIO = H zur Verwendung bei der Behandlung von Krankheiten, ausgewählt aus der Gruppe bestehend aus: Systemischer Sklerose, Morphea, Eosinophiler Fasziitis, Idiopathischer Lungenfibrose, Glomerulonephritis, Sarkoidose, rezidivierender Polychondritis, rheumatoider Arthritis, systemischem Lupus erythematodes, Sjögren-Syndrom, Morbus Crohn, Colitis ulcerosa, atrophische Antrumgastritis, primäre biliäre Zirrhose, Polymyositis, Dermatomyositis, Psoriasis, Psoriasis-Arthritis, Pemphigus, kutane und systemische Vaskulitis, Uveitis, Morbus Behçet, Amyloidose.

2. Pharmazeutische Zusammensetzung, die Folgendes aufweist: 3,3',5,5'-Tetramethoxy[I, I'-biphenyl]-4,4'-diol und pharmazeutisch brauchbare Hilfsstoffe davon zur Verwendung bei der Behandlung von Erkrankungen, ausgewählt aus der Gruppe bestehend aus: Systemischer Sklerose, Morphea, Eosinophiler Fasziitis, Idiopathischer Lungenfibrose, Glomerulonephritis, Sarkoidose, rezidivierender Polychondritis, rheumatoider Arthritis, systemischem Lupus erythematodes, Sjögren-Syndrom, Morbus Crohn, Colitis ulcerosa, atrophische Antrumgastritis, primäre biliäre Zirrhose, Polymyositis, Dermatomyositis, Psoriasis, Psoriasis-Arthritis, Pemphigus, kutane und systemische Vaskulitis, Uveitis, Morbus Behçet, Amyloidose.

3. Pharmazeutische Zusammensetzung zur Verwendung gemäß Anspruch 2 oder pharmazeutische Zusammensetzung, die die Verbindung gemäß Anspruch 1 zur Verwendung gemäß Anspruch 1 aufweist, in der Form von brauchbaren Lösungen, Suspensionen, Pulvern, Granulaten, Tabletten, Pillen, Kapseln, Sirupen, Zäpfchen, Sprays, Aerosolen oder Systemen mit kontrollierter Freisetzung, Creme, Salbe oder Gel, Gleitmitteln, Pasten, Sirupen, Ampullen, Tropfen, Augentropfen, Aerosolen.

4. Pharmazeutische Zusammensetzung zur Verwendung gemäß Anspruch 2 oder pharmazeutische Zusammensetzung, die die Verbindung gemäß Anspruch 1 zur Verwendung gemäß Anspruch 1 aufweist, zur oralen, transdermalen, subkutanen, intravenösen, intramuskulären, rektalen und intranasalen Verabreichung.

## Revendications

1. Composé de formule (1) 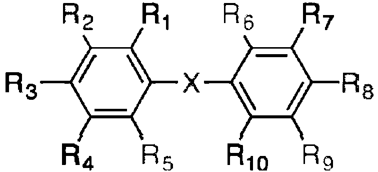 **dans lequel**
X est absent ; R1 = H ; R2 = Pr ; R3 = OH ; R4 = H ; R5 = H ; R6 = H ; R7 = H ; R8 = OH ; R9 = Pr : R10 = H pour utilisation dans le traitement de maladies sélectionnées parmi le groupe consistant en : sclérose systémique, morphée, fasciite à éosinophiles, fibrose pulmonaire idiopathique, glomérulonéphrite, sarcoïdose, polychondrite atrophiante, polyarthrite rhumatoïde, lupus érythémateux systémique,
syndrome de Sjögren, maladie de Crohn, rectocolite hémorragique, gastrite atrophique antrale, cirrhose biliaire primaire, polymyosite, dermatomyosite, psoriasis, arthrite psoriasique, pemphigus, vascularite cutanée et systémique, uvéite, maladie de Behçet, amylose.

2. Composition pharmaceutique comprenant du 3,3',5,5'-tétraméthoxy[1,1'-biphényl]-4,4'-diol ; et des excipients pharmaceutiquement acceptables de celui-ci pour utilisation dans le traitement de maladies sélectionnées parmi le groupe consistant en : sclérose systémique, morphée, fasciite à éosinophiles, fibrose pulmonaire idiopathique, glomérulonéphrite, sarcoïdose, polychondrite atrophiante, polyarthrite rhumatoïde, lupus érythémateux systémique,
syndrome de Sjögren, maladie de Crohn, rectocolite hémorragique, gastrite atrophique antrale, cirrhose biliaire primaire, polymyosite, dermatomyosite, psoriasis, arthrite psoriasique, pemphigus, vascularite cutanée et systémique, uvéite, maladie de Behçet, amylose.

3. Composition pharmaceutique pour utilisation selon la revendication 2 ou composition pharmaceutique comprenant le composé de la revendication 1 pour utilisation selon la revendication 1, sous forme de solutions, suspensions, poudres, granulés, comprimés, pilules, gélules, sirops, suppositoires, pulvérisations, aérosols ou systèmes à libération régulée, crème, pommade ou gel, lubrifiants, pâtes, sirops, flacons, gouttes, gouttes oculaires, aérosols acceptables.

4. Composition pharmaceutique pour utilisation selon la revendication 2 ou composition pharmaceutique comprenant le composé de la revendication 1 pour utilisation selon la revendication 1, à administrer par voie orale, transdermique, sous-cutanée, intraveineuse, intramusculaire, rectale et intranasale.
